# EUROPEAN PATENT APPLICATION

(11) **EP 2 062 527 A1**
(43) Date of publication of application: **27.05.2009**
(21) Application number: 08253544.4
(22) Date of filing: 30.10.2008
(51) Int. Cl.: A61B 5/00

(54) **User interface for insulin infusion device**

(30) Priority: 31.10.2007 US 984059 P
(71) Applicant: Animas Corporation, West Chester, PA 19380 (US)
(72) Inventor: Montgomery, Barbara, Malvern, PA 19355 (US); McLaughlin, Brian J., Media, PA 19063 (US); Brewer, Carl, Ephrata, PA 17522 (US); Destefano, Mark, Collegeville, PA 19426 (US); Shkolnik, Marat, Aston, PA 19014 (US)
(74) Representative: Tunstall, Christopher Stephen

(57) **Abstract**

The application relates to insulin infusion devices (200) and to software employed by such devices to carry out functions of the infusion device. In particular the application relates to a method, comprising: providing an insulin infusion device; providing a blood glucose testing device (205) that is in communication with the insulin infusion device; determining a blood glucose value of a patient; determining a quantity of insulin to deliver to the patient based on the blood glucose value of the patient; delivering the quantity of insulin into the patient; measuring a period of time following the delivery of the quantity of insulin; comparing the period of time to an insulin-absorption curve; determining an amount of insulin absorbed by the patient; and determining an insulin on board amount, wherein the insulin on board amount equals the quantity of insulin delivered to the patient less the amount of insulin absorbed by the patient.

## Description

### FIELD OF THE INVENTION

The present invention relates, in general, to insulin infusion devices and, more particularly, to software employed by such devices to carry out functions of the infusion device and to generate screen displays forming a user interface.

### BACKGROUND OF THE INVENTION

Diabetes mellitus is a chronic metabolic disorder caused by an inability of the pancreas to produce sufficient amounts of the hormone insulin so that the metabolism is unable to provide for the proper absorption of sugar and starch. This failure leads to hyperglycemia, i.e. the presence of an excessive amount of glucose within the blood plasma. Persistent hyperglycemia causes a variety of serious symptoms and life threatening long term complications such as dehydration, ketoacidosis, diabetic coma, cardiovascular diseases, chronic renal failure, retinal damage and nerve damages with the risk of amputation of extremities. Because healing is not yet possible, a permanent therapy is necessary which provides constant glycemic control in order to always maintain the level of blood glucose within normal limits. Such glycemic control is achieved by regularly supplying external insulin to the body of the patient to thereby reduce the elevated levels of blood glucose.

External insulin was commonly administered by means of typically one or two injections of a mixture of rapid and intermediate acting insulin per day via a hypodermic syringe. While this treatment does not require the frequent estimation of blood glucose, it has been found that the degree of glycemic control achievable in this way is suboptimal because the delivery is unlike physiological insulin production, according to which insulin enters the bloodstream at a lower rate and over a more extended period of time. Improved glycemic control may be achieved by the so-called intensive insulin therapy which is based on multiple daily injections, including one or two injections per day of long acting insulin for providing basal insulin and additional injections of rapidly acting insulin before each meal in an amount proportional to the size of the meal. Although traditional syringes have at least partly been replaced by insulin pens, the frequent injections are nevertheless very inconvenient for the patient

Substantial improvements in diabetes therapy have been achieved by the development of the insulin infusion pump relieving the patient of the daily use of syringes or insulin pens. The insulin pump allows for the delivery of insulin in a more physiological manner and can be controlled to follow standard or individually modified protocols to give the patient a better glycemic control over the course of a day.

Infusion pumps can be constructed as an implantable device for subcutaneous arrangement or can be constructed as an external device with an infusion set for subcutaneous infusion to the patient. External infusion pumps are mounted on clothing, hidden beneath or inside clothing, or mounted on the body. Implanted pumps are controlled by a remote device. Most external infusion pumps are controlled through a built-in user interface, but control via a remote controller is available for some pump systems. Some pump systems use both a built-in pump user interface and a remote controller.

Regardless of the type of infusion pump, blood glucose monitoring is still required for glycemic control. For example, delivery of suitable amounts of insulin by the insulin pump requires that the patient frequently determines his or her blood glucose level and manually input this value into the remote device or into the built in user interface for some external pumps, which then calculates a suitable modification to the default or currently in use insulin delivery protocol, i.e. dosage and timing, and subsequently communicates with the insulin pump to adjust its operation accordingly. The determination of blood glucose concentration is performed by means of a suitable battery-operated measuring device such as a hand-held electronic meter which receives blood samples via enzyme-based test strips and calculates the blood glucose value based on the enzymatic reaction.

The meter device is an integral part of the blood glucose system and integrating the measuring aspects of the meter into an external pump or the remote of a pump is desireable. Integration eliminates the need for the patient to carry a separate meter device, and it offers added convenience and safety advantages by eliminating the manual input of the glucose readings.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 depicts an external infusion pump system of an embodiment of the invention.

Figure 2 depicts a meter controller and external infusion pump system of an embodiment of the invention.

Figure 3 depicts an external infusion pump with an integrated meter of an embodiment of the invention.

Figure 4 depicts a block diagram showing an illustrative control system for an infusion pump according to an embodiment of the invention.

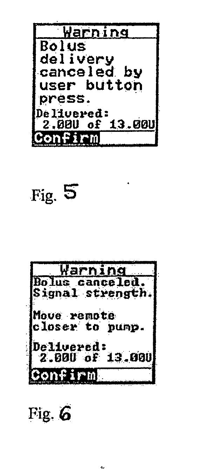

Figure 5 illustrative screen displays which may be displayed by a display screen incorporated into an infusion pump of an embodiment of the invention.

Figure 6 is an exemplary communication failure screen according to one aspect of the invention.

Figure 7 is a flowchart of an embodiment of the motor rotational ticks calculation.

Figure 8 is a flowchart of an alternative embodiment of the motor rotational ticks calculation.

### DETAILED DESCRIPTION OF ILLUSTRATIVE EMBODIMENTS OF THE INVENTION

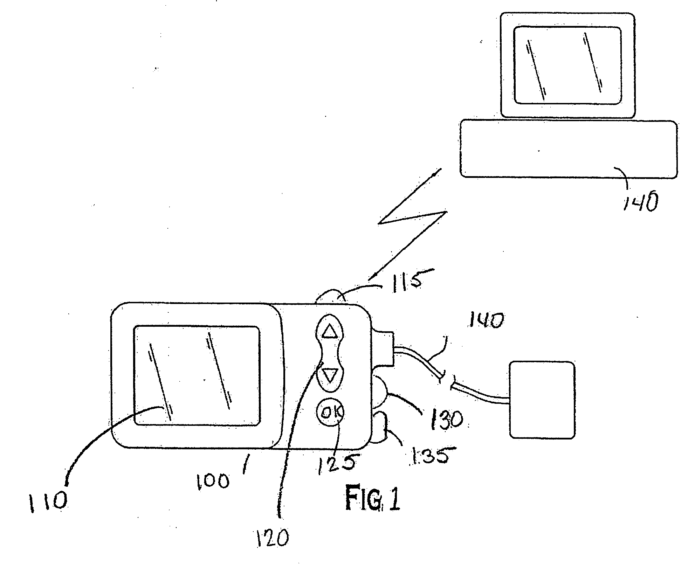

An embodiment of the present invention is depicted in Fig.1. An external infusion pump **100** may be an ambulatory infusion pump that can deliver insulin through an infusion set **140**, permitting subcutaneous infusion of the desired medicine. Although the present illustrative embodiment of the invention relation to the infusion of insulin, other medicines can be infused in this or other, alternative embodiments of the invention. Features of the pump **100** may include, without limitation, basal programs, bolus delivery programs, bolus calculation estimators, limit alarms, reminders, visual, vibratory and auditory alarm indications, pump operation logging, and optionally, a food database to assist in calculating meal carbohydrate amounts. Illustratively, the pump **100** may communicate via a cable or wirelessly to a personal computer ("PC") **140** to upload pump **100** data and download of configuration settings and personal data from the PC **140** to the pump **100**. The PC may include software for maintaining or storing logs, displaying pump data in text or graphical format and may provide analysis to the user and/or healthcare professionals. In the present embodiment, the PC **140** communicates wirelessly to the pump **100** using infra-red ("IR") communications although other wireless technologies such as near or far field radio-frequency may also be used.

Power to the pump **100** can be supplied by a standard lithium or alkaline AA battery located inside of the pump **100**. As shown in the illustration, the power source may be located behind the battery cap **135**. The pump **100** generally includes a display screen **110** for displaying information to the user in the form of a user interface. So that the pump user may interact with the user interface, control devices, such as buttons, are included in the construction of the pump **100**. The present embodiment shows up and down arrow buttons **120**, an "OK" selection button **125**, a user configurable bolus button **130** and a screen adjustment button **115**. In an embodiment of the present invention in which the pump has a display screen **110** with high visibility, the display screen **110** is a color Organic Light Emitting Diode (OLED) although in an alternative embodiment the display is a Liquid Crystal Display (LCD). Means for adjusting the display screen **110** properties may also be include, such as an adjustment button **115** for adjusting the contrast and intensity of the screen **110**.

The pump home screen displays the time, battery level, insulin level and current delivery information, and provides access to the menu-driven user interface and status screens summarizing major pump operations such as basal activity, bolus activity, daily delivery totals, combo bolus activity, temporary basal activity and pump configuration codes. The arrow buttons **120** provide navigation to selectable screen items. The "OK" selection button **125** selects the highlighted screen item. For selected menu items, a sub-menu may be displayed depending on the menu item selected or a destination screen such as a bolus, basal, configuration or history may be displayed. For selected editable items such as the pump time, edit mode is entered and the item blinks indicating the item value is adjustable via up and down arrow buttons **120**. Pressing the "OK" selection button **125** exits edit mode.

In another embodiment of the present invention, a remote controller wirelessly commands the pump **100** of Fig.1. The wireless communication is via far-field radio frequency. In alternative embodiments, infra-red, near-field radio frequency or intra-body communication provide wireless communication. In another embodiment of the present invention, the remote control user interface includes a display screen, up and down buttons, "OK" selection button, user configurable bolus button and screen adjustment button. For patients concealing the pump **100** under clothing, the remote controller maintains patient privacy. Parents and caregivers of a young child pump patient benefit from the remote by avoiding the need to wake a sleeping child for pump operation.

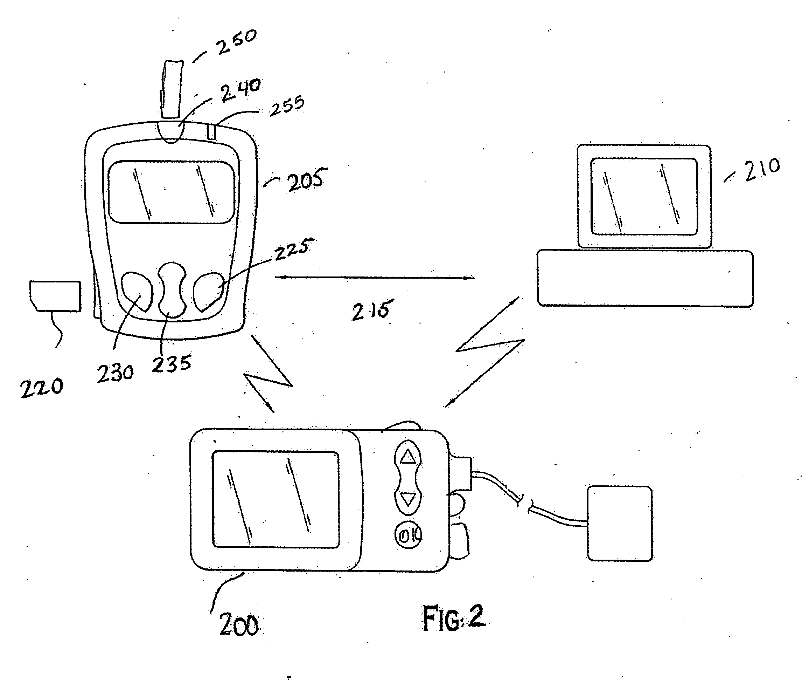

A variation of the remote controlled pump is illustrated in Fig. 2. In this embodiment, the external infusion pump **200** communicates wirelessly with an integrated blood glucose meter and remote controller **205**. The meter controller **205** is a meter and strip system for measurement of whole blood glucose with a disposable test strip **250**. Meter controller **205** accepts the test strip **250** inserted in the test port **240**. Except as noted, the pump **200** features include the pump **100** features of Fig.1. Optionally, the pump **200** communicates with the PC **210** as described in connection with the PC **140** of Fig. 1. The blood glucose meter controller **205** optionally communicates with the PC **210** via a universal serial bus (USB) wired connection **215**.

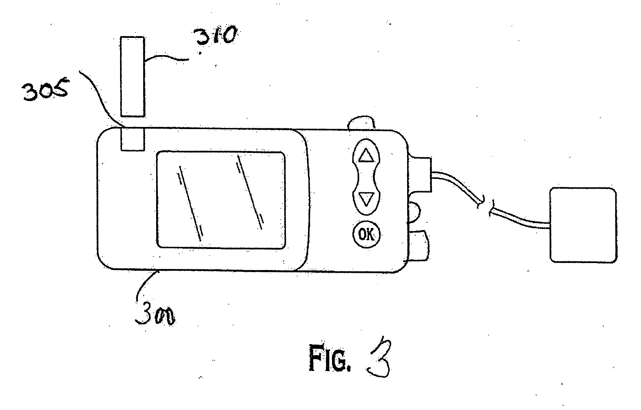

In another embodiment depicted in Fig.3, the integrated pump meter **300** is comprised of an integrated meter and strip system, and, except as noted, the pump **300** includes the features of the pump **100** of Fig. 1. The meter and strip system measures whole the blood glucose from disposable test strip **310** inserted into test port **305**. Optionally, the pump **300** communicates with a PC, not shown, as described in connection with the PC **140** of Fig. 1.

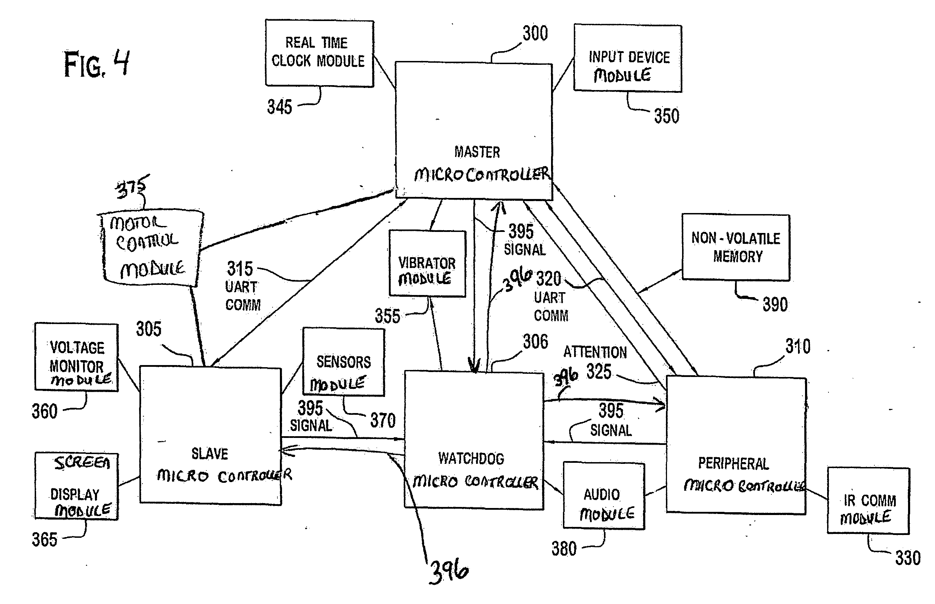

Block diagram Fig. 4 illustrates one embodiment of the pump **100** of Fig.1. Pump control is managed by four microcontrollers: master **400**, slave **405**, peripheral **410** and watchdog **406**. Non-volatile memory and random access memory (RAM) are internal to each microcontroller. The master **400,** slave **405** and peripheral **410** periodically output a pattern via the signals **495** to the watchdog **406** as a check for proper microcontroller operation. Conversely, the watchdog **406** periodically outputs a pattern via the signals **496** back to all other microcontrollers.

Operational modules, such as the real time clock module **445**, are controlled by one or more microcontrollers. In alternative embodiments, a single microcontroller or additional microcontrollers operate the pump **100**. In another alternative embodiment, microprocessors or microcontrollers with external memory control the pump **100**. In yet another embodiment, operational modules are arranged differently and controlled by other microcontrollers and/or microprocessors.

Referring again to Fig. 4, serial message passing is the primary inter-microcontroller communication path between master **400**, slave **405** and peripheral **410** microcontrollers. The master **400** acts as the communication master and sends requests to the slave **405** and peripheral **410** over the bidirectional universal asynchronous receiver transmitter (UART) communications **415** and **420** respectively. The master **400** receives message responses from the targeted microcontroller via the same communication path. Each microcontroller on these communication paths monitors message traffic to ensure the receiving microcontroller is operating properly.

The peripheral **410** uses a unidirectional line **425** to demand master **400** communication attention. The non-volatile memory **490** stores language specific strings, settings and logged pump data using serial bus **440**. Memory **490** is comprised of two serial electrically erasable programmable read only memories (SEEPROM) although in alternative embodiments a single non-volatile memory or additional memories, or a different non-volatile memory technology are used. In the present embodiment, the memory **490** is accessed via an I2C bus **440**. In an alternative embodiment, the memory **490** is accessed via a system bus or other serial interface.

The master **400** manages the storage **490** during end-user pump operation. The master **400** controls the real-time clock module **445** that as serves as the pump timekeeper. The input device module **450** interfaces to the up and down arrow buttons **120**, the "OK" selection button **125**, the user configurable bolus button **130** and the screen adjustment button **115** of Fig. 1. Except during a watchdog fault, the master **400** controls the vibrator module **455**. The master **400** manages the overall pump operation including, without limitation, inter-microcontroller message communication, infusion delivery amount estimation, pump delivery oversight, local user requests and in a remotely operated pump as in Fig. 2, remote user requests. In the event of a pump error or failure, the master **400** halts pump delivery by powering off the motor control module **475**.

The slave **405** also services the master **400** message requests for the voltage monitor module **460** status, the screen display module **465** rendering and setting changes, the sensors module **470** status, the motor control module **485** and some delivery computations. The slave **405** operates the drive mechanism. In a preferred embodiment, the slave **405** applies force to a removable tubular cartridge reservoir and linear plunger by activating a DC motor via the motor control module **485**. The motor turns a lead screw applying pressure to the plunger and forcing the infusion medium through the infusion set to the patient. The slave **405** monitors a force sensor to detect occlusions and periodically, the slave **405** reads the Hall Effect sensors to determine motor direction and incremental motor rotation. The smallest rotary movement is one tick.

The peripheral **410** controls audio operation through the audio module **480**. IR messages are sent and received by the peripheral **410** using the IR comm module **430**. In a remote controlled pump embodiment or a meter controller embodiment such as Fig. 2, the peripheral **410** sends and receives RF messages via a wireless communication module, not shown. In an integrated pump meter embodiment as in Fig. 3, the peripheral **410** uses a serial peripheral interface bus (SPI) to send and receive message from the integrated meter module, not shown. In an alternative embodiment, the peripheral **410** uses a UART bidirectional serial bus to communicate with the meter module.

The external pump **100** basal insulin deliveries are used to maintain a steady level of insulin over a certain period of time. Bolus deliveries compensate for significant increases in blood glucose attributable to meals, activities and correction to blood glucose (BG) readings. Basal programs are user configurable profiles comprising at least one segment where each segment contains a start time and a level of infusion to start at that time and in effect until the next segment start time or the end of the day when the program is restarted. In the present embodiment, one to four basal programs each providing 12 segments are supported, but in alternative embodiments more programs and /or segments are available. Multiple basal programs allow the user to accommodate schedules with differing levels of activities such as work days, sick days, weekends and exercise days. For prolonged activity variations, a temporary basal adjustment is applied to the current basal program. The user specifies a +/- percentage of the current basal amount and the duration of the temporary basal.

The present invention supports several bolus delivery types and several bolus commands including some commands with bolus estimation calculators. Bolus delivery types include a normal delivery where the specified infusion amount is delivered immediately and a combo delivery comprised of two portions: normal and extended. The normal portion is delivered immediately with the extended portion delivered over a user configurable period of time. The user adjusts the combo bolus distribution of normal and extended portions from 0 %to 100% although some bolus commands recommend a preferred distribution.

A normal bolus command delivers the user selected infusion amount using the normal delivery type. A combo bolus command delivers the user selected infusion amount using the combo delivery type. An auto bolus command permits the user to initiate a bolus without the need to look at the pump screen. Auto bolus delivers increments of a user configurable infusion amount using the normal delivery type. The infusion amount is incremented with each press of the bolus button. Based on the auto bolus indication setting, the pump will vibrate or beep for each button press then wait for a period of time without a button press and vibrate or beep once for each button press to confirm the count. Finally, the pump vibrates or beeps before delivering the infusion amount.

The bolus commands with bolus estimation calculators may employ personal profiles for data such as target BG ranges, insulin sensitivity factor (ISF) and insulin to carbohydrate (I:C) ratios. Each personal profile holds up to 12 segments but in other embodiments additional segments are available. Each segment contains a start time and at lease on associated data setting in effect until the next segment time or the end of the day when the profile is restarted. For the insulin sensitivity factor and insulin to carbohydrates ratios profile, the data setting is the respective factor or ratio. For the target BG range personal profile, the data setting is a target BG level and a specified +/range around that target BG level.

The estimation calculators also account for Insulin-On-Board (IOB). IOB is the insulin delivered to the patient but not yet metabolized into the body. It is calculated based on an absorption curve of fast-acting insulin and updated periodically.

The bolus command, for example ezBG as employed in an infusion pump sold by Animas Corp. of West Chester, PA, calculates the estimated infusion amount based on an entered actual BG reading, the current target BG range, the current ISF value and the IOB. The estimate is displayed for the user, but the user selects the delivery amount. This delivery amount is then delivered using the normal delivery type.

An entered actual BG reading and/or carbohydrates can be entered into a carbohydrate calculator, such as ezCarb which is employed by an infusion device sold by Animas Corp. of West Chester, PA, which then uses this information in a bolus command calculator. Carbohydrates are entered directly, and on systems with a food database, users select food items from a list, specify the serving sizes and the carbohydrates are summed by the calculator. The infusion amount to compensate for carbohydrates uses the current I:C. To compensate for the BG reading, the current ISF value and the target BG range along with any IOB are used to estimate the infusion amount. The estimate is displayed for the user, but the user selects the delivery amount and either a normal or combo type delivery.

The pump 100 provides user adjustable delivery limits to prevent over infusion. Delivery limits may include a one hour maximum basal limit, a total daily delivery dose limit, a two hour limit and a bolus limit. Exceeding a limit often triggers a pump alarm that must be acknowledged by the user before normal pump operation resumes. For some bolus commands, the bolus limit prohibits the user from selecting a bolus delivery amount exceeding the limit setting.

The pump 100 indicates delivery notifications, alerts, reminders, warnings and alarms to the user. The screen 100 displays the event but certain indications are accompanied by auditory or vibratory indications. Depending on the specific event, display only, vibratory or auditory indications are user configurable settings. For example, after each auto bolus button press, the pump can vibrate or put out an audio beep at low, middle or high volume, certain errors and alarms automatically progress to vibratory indications, louder auditory indications or both. In a remote controlled pump such as in Fig. 2, indications are also propagated to the remote. Many of these indications can be confirmed and acknowledged on the remote thereby clearing the pump **200** indication as well.

Referring again to Fig. 1, the pump 100 logs pump activity for history purposes and for pump failure analysis. Logged data includes errors, alarms and warnings, total daily dose information, prime events, suspend events, cartridge rewind, alignment, power-on restarts, settings reset, time/date changes, blank basal programs, active basal program, all infusion deliveries, force sensor and voltage readings. In a preferred embodiment of the present invention, history records are stored in memory based on the record type for faster data retrieval. Remotely controlled pumps similarly log pump activities remotely initiated. In a pump remotely controlled by a meter controller such as Fig. 2, the pump logs blood glucose readings from the meter controller. This logging consolidates the data in the event that the meter controller is forgotten or inoperable when health care advice is necessary.

For large infusion deliveries, such as an insulin bolus, the requested infusion amount is broken down into smaller delivery portions such as units. As each portion is delivered, the delivery amount is recorded in the pump history until the entire amount is delivered or the delivery is prematurely terminated. A delivery is terminated for several reasons. For example in the pump embodiment of Fig. 1, the user terminates the delivery after pressing the auto bolus button **130** too many times or entering the incorrect bolus amount. In a remote controlled pump embodiment such Fig. 2, an automatic delivery termination occurs upon certain communication failures between the remote and the pump.

When a delivery is terminated, the recorded pump history indicates the delivered infusion amount and the initial requested delivery amount. The user accesses these history records to review the delivery details. In addition, the bolus status screen displays the current IOB and details of the last delivered amount. Displaying the delivered infusion of a terminated delivery whether by accessing the pump history or the bolus status screen requires confirming the termination warning, navigating to the main menu to select the type of desired data (e.g. History or Status) then selecting the history record or status screen with the desired information.

In the embodiment of Fig.1, the user navigates via the pump user interface and the data is displayed on the pump display. In alternative embodiments with a remote controller or meter controller as in Fig. 2, the controller is used to navigate and display the data. Regardless of the device used to view the delivery information, eliminating the navigation and selection steps to view the delivered and targeted delivery amounts makes the infusion system easier to use.

Figs. D and E depict two embodiments of the present invention that eliminates the additional navigation and selection steps required to view the History and Status data upon delivery termination. The screen of Fig. D could appear on the pump of the Fig. 1, or the remote controller or meter controller of Fig. 2. Fig. D shows the cause of the termination, a user button press together with the infusion amount delivered and the requested infusion amount. Presenting this infusion data on the termination screen eliminates the need to view the history or status screens and presents the data at a more desirable and timely location.

Fig. E shows an embodiment of the present invention where a communication failure occurs. This screen occurs in a remote operated system such as the system embodiment of Fig. 2. With a communication failure, the pump has the most up-to-date delivery information and displays the termination screen. The screen shows the cause of the termination, the infused delivery amount and requested infusion amount and optionally a recommended action to fix the termination cause.

Referring to again Fig. 4, infusion delivery requests are sent by the master **400** to the slave **405** over the UART communications **415** for delivery. The delivery request amount is specified in units although in alternative embodiments, a different unit of measure can be used or the master **400** may request a whole number of motor encoder ticks. In a preferred embodiment, the slave **405** converts the requested delivery units into a whole number of motor encoder ticks then adds whole ticks when the fractional ticks from previous deliveries form a whole tick. The whole ticks count is adjusted by the number of error whole ticks from previous deliveries. The count is then checked against a minimum tick count. If delivery ticks remain, the slave **415** initiates delivery of the infusion amount.

In a preferred embodiment of the present invention, the conversion step may use an accumulator holding a high resolution fractional motor rotation ticks format to simplify computational complexity. The accumulated infusion amount holds undelivered fractional ticks from earlier delivery requests and the latest infusion request.

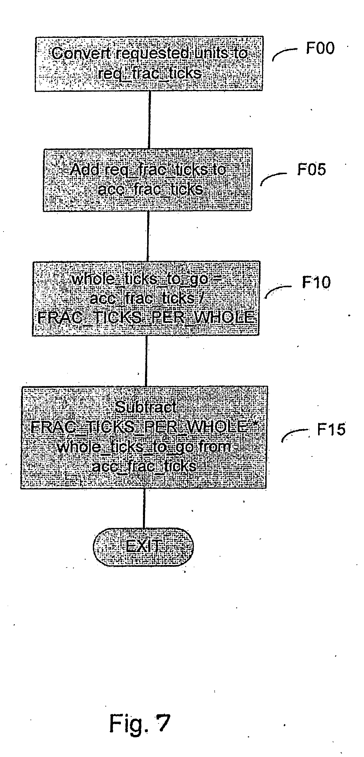

One embodiment of the conversion step is illustrated in the flowchart of Fig. 7. In step **F00,** the requested infusion amount, specified in units, is converted to high resolution fractional ticks. At step **F05**, the requested fractional ticks are added to the accumulator of fractional ticks. The whole tick count required to control motor operation is computed at step **F10** by dividing the accumulated fractional ticks by the number of fractional ticks per whole tick. At step **F15**, the accumulated fractional ticks are reduced by the product of the number of fractional ticks per whole tick and the whole ticks count.

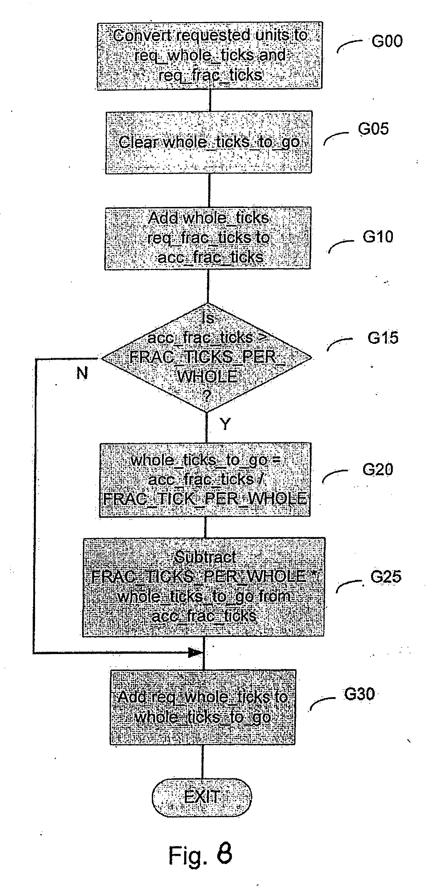

Fig. 8 illustrates an alternative embodiment of the conversion step. At step **G00,** the requested units are converted into requested whole ticks and requested fractional ticks. At step G05, the whole ticks count is set to zero. The requested fractional ticks are added to the accumulated fractional ticks at step **G10**. The test at step **G15** determines if whole ticks can be formed from the accumulated fractional ticks by comparing against the number of fractional ticks in a whole tick. If not, proceed to step **G30.** Otherwise, the whole tick count attributable to fractional ticks is computed at step **G20** by dividing the accumulated fractional ticks by the number of fractional ticks per whole tick. At step **G25**, the accumulated fractional ticks are reduced by the number of fractional ticks per whole tick multiplied by the whole ticks. The final calculation of the whole tick count is computed at step **G30**, by adding the requested whole tick counts to the whole tick count.

Once the count of whole ticks is computed, the count is adjusted by the error ticks count. Events such as motor overshoot or undershoot, and tick counts below a minimum tick limit are stored in the error tick accumulator. Next, the whole ticks count is checked against the minimum tick limit. This check prevents small infusion deliveries that cannot be accurately delivered by the pump system. In the case of such as request, the whole ticks count is added to the error tick count and no delivery initiated.

Otherwise, referring again to Fig.4, the motor control module **475** is activated, the force sensor of sensor module **470** is checked for an occlusion and the Hall effect sensors of sensor module **470** are initialized. Periodically, the slave **405** checks the Hall effect sensors to determine the motor direction and position, and adjust the whole tick count accordingly. When no additional whole delivery ticks remain, the motor is stopped and the delivery is completed.

It will be recognized that equivalent structures may be substituted for the structures illustrated and described herein and that the described embodiment of the invention is not the only structure which may be employed to implement the claimed invention. In addition, it should be understood that every structure described above has a function and such structure can be referred to as a means for performing that function. While embodiments of the present invention have been shown and described herein, it will be obvious to those skilled in the art that such embodiments are provided by way of example only. Numerous variations, changes, and substitutions will now occur to hose skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method, comprising:
providing an insulin infusion device;
providing a blood glucose testing device that is in communication with the insulin infusion device;
determining a blood glucose value of a patient;
determining a quantity of insulin to deliver to the patient based on the blood glucose value of the patient;
delivering the quantity of insulin into the patient;
measuring a period of time following the delivery of the quantity of insulin;
comparing the period of time to an insulin-absorption curve;
determining an amount of insulin absorbed by the patient; and
determining an insulin on board amount, wherein the insulin on board amount equals the quantity of insulin delivered to the patient less the amount of insulin absorbed by the patient.

2. The method of claim 1, comprising selecting a quantity and type of food from a food database stored in the insulin infusion device.

3. The method of claim 2, comprising determining a carbohydrate content of the quantity and type of food.

4. The method of claim 3, wherein the quantity of insulin to deliver to the patient is determined based upon the blood glucose value of the patient and the carbohydrate content of the quantity and type of food.
